# EUROPÄISCHE PATENTSCHRIFT

(11) **EP 2 103 326 B1**
(45) Veröffentlichungstag und Bekanntmachung des Hinweises auf die Patenterteilung: **15.06.2011**
(21) Anmeldenummer: 09153311.7
(22) Anmeldetag: 20.02.2009
(51) Int. Cl.: A61N 1/05, A61N 1/36, A61B 8/12

(54) **Hirnstimulationselektrodenleitung sowie Einführungsvorrichtung**
Brain stimulation electrode lead and introduction device
Sonde pour électrodes de stimulation du cerveau et dispositif d'introduction

(30) Priorität: 20.03.2008 DE 102008015156
(43) Veröffentlichungstag der Anmeldung: 23.09.2009
(73) Patentinhaber: Biotronik CRM Patent AG, 6341 Baar (CH)
(72) Erfinder: Dörr, Thomas, 12437, Berlin (DE)
(74) Vertreter: Lindner-Vogt, Karin L.

(56) Entgegenhaltungen:
- EP-A- 1 062 973
- WO-A-2006/116256
- US-A1- 2006 047 333

## Beschreibung

Die Erfindung betrifft eine Hirnstimulationselektrodenleitung zum Stimulieren tief liegender Hirnbereiche. Die Hirnstimulationselektrodenleitung besitzt einen längsgestreckten biegsamen Elektrodenleitungskörper, an dessen distalem Ende oder in der Nähe von dessen distalem Ende wenigstens eine Stimulationselektrode angeordnet ist. Diese Stimulationselektrode dient im Einsatzfall der Abgabe elektrischer Impulse an umgebendes Körpergewebe.

Die Stimulation tief liegender Hirnbereiche ist eine medizinische Behandlungsmethode, mit der sich beispielsweise Symptome der Parkinson'schen Krankheit behandeln lassen. Üblicherweise werden derzeitig Hirnstimulationselektroden für die Deep Brain Stimulation (DBS) mittels einer neuroradiologischen Planung auf Basis von Computertomographie- und/oder Magnetresonanztomographie-Daten implantiert. Die Hirnstimulationselektrodenleitungen können derzeit nur in Geradeausrichtung mit Hilfe eines stereotaktischen Zielbügels vorgeschoben werden. Die Implantationsrichtung wird dabei entsprechend der neuroradiologischen Planung derart bestimmt, dass die mögliche Verletzung interkranieller Blutgefäße vermieden wird. Eine derartige Elektrode wird in der EP 1 062 973 A1 beschrieben.

Ein Nachteil dieses Verfahrens ist das verbleibende Restrisiko einer intrakraniellen Blutung, weil beispielsweise das Gehirn bezüglich der geplanten Koordinaten während der Implantation verschoben sein kann.

Ein weiterer Nachteil der beschriebenen bekannten Implantationstechnik besteht darin, dass auf ihrer Basis keine steuerbaren Hirnstimulationselektrodenleitungen implantiert werden können, da in diesem Fall die neuroradiologische Planung nicht für alle mit der steuerbaren Hirnstimulationselektrodenleitung möglichen Implantationswege in Echtzeit durchgeführt werden kann.

Der Erfindung liegt die Aufgabe zugrunde, eine Hirnstimulationselektrodenleitung anzugeben, mit der sich wenigstens einige der Nachteile des Standes der Technik vermeiden lassen.

Erfindungsgemäß wird diese Aufgabe durch eine Hirnstimulationselektrodenleitung entsprechend Anspruch 1 gelöst.

Gegebenenfalls können auch mehrere Ultraschalltransducer vorgesehen sein, die so angeordnet sind, dass sie zum Teil in gerader Richtung der Elektrodenleitung vorausschauen und teilweise schräg vorausschauen.

Der oder die Ultraschalltransducer sind vorzugsweise Pulse Wave Doppler Sensoren zum Nachweis bewegter Erythrozyten.

Vorzugsweise ist die Hirnstimulationselektrodenleitung mit einer Einführvorrichtung verbunden, die vorzugsweise wenigstens einen Ultraschallgenerator und -empfänger sowie einen Demodulator aufweist, die Ultraschalltransducer zur Abgabe von Ultraschallwellen anzusteuern und die von den Ultraschalltransducern infolge empfangener reflektierter Ultraschallwellen generierten Signale zu empfangen und zu demodulieren. Der Demodulator ist mit einer Steuer- und Auswerteeinheit verbunden, die ausgebildet ist, aus dem demodulierten Ultraschallsignal ein Erfassungssignal zu erzeugen, das anzeigt, ob sich ein Blutgefäß im Erfassungsbereich des Ultraschalltransducers befindet.

Dabei kann die Steuer- und Auswerteeinheit entweder mit einer optischen Anzeige oder mit einem akustischen Signalgeber oder beidem verbunden sein.

Vorzugsweise ist die Steuer- und Auswerteeinheit in Verbindung mit der optischen Anzeige oder dem akustischen Signalgeber oder beiden so ausgebildet, dass das angezeigte oder akustisch wiedergegebene Signal je nach Abstand eines erfassten Blutgefäßes vom jeweiligen Ultraschalltransducer unterschiedlich ist. Die optische Anzeige ist in diesem Zusammenhang vorzugsweise so ausgebildet, dass die Entfernung eines erfassten Blutgefäßes zum jeweiligen Ultraschalltransducer durch unterschiedliche Farben dargestellt wird. Im Falle des akustischen Signalgebers ist die Steuer- und Auswerteeinheit in Verbindung mit diesem akustischen Signalgeber vorzugsweise dazu ausgebildet, unterschiedliche Entfernungen zwischen einem erfassten Blutgefäß und einem jeweiligen Ultraschalltransducer durch unterschiedliche Tonhöhen wiederzugeben.

Vorzugsweise umfasst die Hirnstimulationselektrodenleitung nicht nur einen, sondern mehrere Transducer, beispielsweise fünf Ultraschalltransducer, von denen einer einen Erfassungsbereich besitzt, der exakt entlang der verlängerten Längsachse des Elektrodenleitungskörpers ausgerichtet ist, während vier weitere Ultraschalltransducer so ausgerichtet sind, dass sie jeweils unterschiedliche, schräg seitlich ausgerichtete Sektoren erfassen.

In diesem Fall umfasst die Einführvorrichtung vorzugsweise einen Kanalumschalter, mit dessen Hilfe der Ultraschallgenerator und -empfänger sowie der Demodulator wahlweise mit einem der verschiedenen Ultraschalltransducer verbunden werden kann. Die Ausgangssignale der verschiedenen Ultraschalltransducer werden dann reihum wechselseitig nach einer Art Zeit-Multiplexverfahren verarbeitet, indem jeweils der Kanalumschalter umgeschaltet wird. Alternativ könnte auch jeder Ultraschalltransducer mit einem eigenen Ultraschallgenerator und -empfänger verbunden sein. Dies würde jedoch den Aufwand unnötig erhöhen. Im Falle einer Mehrzahl von Ultraschalltransducern und eines Kanalumschalters ist die Steuer- und Auswerteeinheit vorzugsweise mit dem Kanalumschalter verbunden und bewirkt das Umschalten der Verbindung zwischen dem einen Ultraschallgenerator und -empfänger und den verschiedenen Ultraschalltransducern.

Im Falle einer Mehrzahl von Ultraschalltransducern ist die Steuer- und Auswerteeinheit außerdem ausgebildet, auf der optischen Anzeige nicht nur ein Signal anzuzeigen, welches die Nähe eines Blutgefäßes in farbcodierter Weise wiedergibt, sondern welches zusätzlich auch die Lage eines erfassten Blutgefäßes relativ zur verlängerten Längsachse des distalen Endes des Elektrodenleitungskörpers wiedergibt. Beispielsweise kann die optische Anzeige so aufgebaut sein, dass sie in wenigstens fünf Anzeigesektoren unterteilt ist, von denen ein zentraler Sektor von vier peripheren Sektoren umgeben ist. In dem zentralen Sektor wird jeweils ein aus den Ausgangssignalen des zentralen Ultraschalltransducers gewonnenes Anzeigesignal wiedergegeben. Die übrigen vier Anzeigesektoren sind einem oder mehreren jeweils peripheren Ultraschalltransducern zugeordnet. Gemäß dieser letztgenannten Ausführungsvariante kann beim Einführen der erfindungsgemäßen Hirnstimulationselektrodenleitung nicht nur der Abstand zu einem Blutgefäß berücksichtigt werden, sondern zusätzlich auch noch die relative laterale Lage eines erfassten Blutgefäßes. Dies erlaubt ein gezieltes Ausweichen beim Einführen des Hirnstimulationskatheters und somit in noch sicherer Weise die Vermeidung intrakranialer Blutungen.

Vorzugsweise weist die Hirnstimulationselektrodenleitung weniger als 15 Ultraschalltransducer auf.

Die Erfindung soll nun anhand eines Ausführungsbeispiels mit Bezug auf die Figuren näher erläutert werden. Von den Figuren zeigt:
- Fig. 1:: einen schematischen Längsschnitt durch ein distales Ende einer erfindungsgemäßen Hirnstimulationselektrodenleitung;
- Fig. 2a und 2b:: einen schematischen Längsschnitt durch ein distales Ende einer alternativen Ausführungsvariante einer Hirnstimulationselektroden- leitung;
- Fig. 3a und 3b:: Details einer Ausführungsvariante einer Hirnstimulationselektroden- leitung mit einer Mehrzahl von Ultraschalltransducern in schemati- scher Darstellung;
- Fig. 4:: eine Prinzipdarstellung für eine Einführvorrichtung für eine Hirnsti- mulationselektrodenleitung 10 gemäß der Erfindung; und
- Fig. 5a und 5b:: eine Darstellung einer optischen Anzeige für die Einführvorrichtung gemäß Fig. 3.

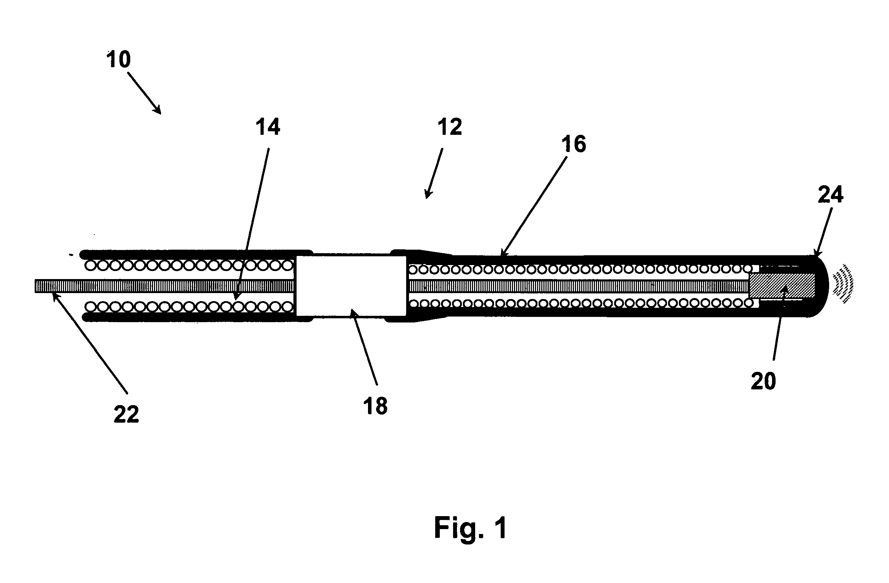

In Fig. 1 ist schematisch ein distales Ende einer Hirnstimulationselektrodenleitung 10 in teilweise geschnittener Darstellung abgebildet. Die Hirnstimulationselektrodenleitung 10 besitzt einen Elektrodenleitungskörper 12, der von einer äußeren Helixwendel 14 aus Metall sowie einer Hülle 16 aus biokompatiblem Kunststoff gebildet wird, die die metallische Helixwendel 14 über den größten Teil der Länge der Hirnstimulationselektrodenleitung 10 umgibt. In der Nähe des distalen Endes der Hirnstimulationselektrodenleitung 10 ist eine Ringelektrode 18 angeordnet, die nicht von der Hülle 16 umgeben ist und eine elektrisch leitende äußere Oberfläche besitzt, die mit der metallischen Helixwendel 14 elektrisch leitend verbunden ist und die der Abgabe von Stimulationsimpulsen dient.

Im Inneren der Hirnstimulationselektrodenleitung 10 ganz an deren distalem Ende ist ein Ultraschalltransducer 20 angeordnet, der über wenigstens eine Signalleitung 22 mit einem in Fig. 1 nicht dargestellten Anschluss der Hirnstimulationselektrodenleitung verbunden ist.

Der Ultraschalltransducer 20 ist in einen Abschlusskörper 24 eingebettet, der die Hirnstimulationselektrodenleitung 10 bzw. deren Elektrodenleitungskörper 12 an seinem distalen Ende verschließt. Der Abschlusskörper 24 besteht aus einem Material, welches biokompatibel ist und außerdem ein Ein- und Auskoppeln zum Übertragen von Ultraschallwellen mit geringer Dämpfung erlaubt. Die Form des Abschlusskörpers ist an die Form des Ultraschalltransducers 20 so angepasst, dass sich der Ultraschalltransducer 20 und der Abschlusskörper 24 großflächig berühren.

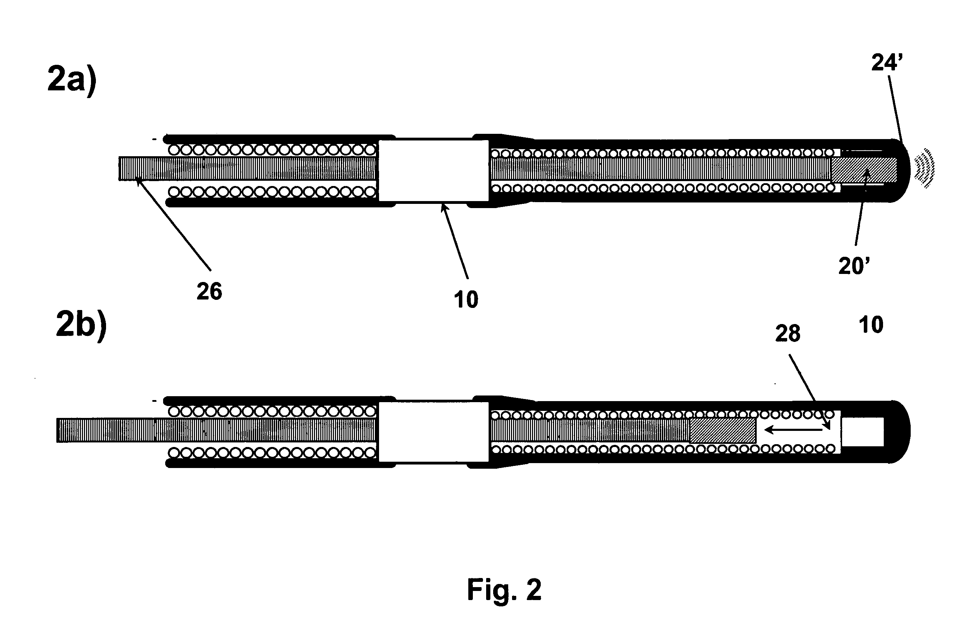

In Figuren 2a und 2b ist eine bevorzugte Ausführungsvariante dargestellt, bei der der Ultraschalltransducer 20' und die Signalleitung 22' in einem Lumen des Elektrodenleitungskörpers 12 längsbeweglich geführt sind. Die Signalleitung 22' ist dazu als entfernbarer Mandrin ausgebildet. Fig. 2a zeigt den Ultraschalltransducer 20' und die Signalleitung 22' in ihrem vollständig in das Lumen des Elektrodenleitungskörpers 12' eingesetzten Zustand, bei dem wenigstens eine Frontfläche des Ultraschalltransducers 20' bündig an einer Innenfläche des Abschlusskörpers 24' anliegt. Fig. 2b zeigt, wie der Ultraschalltransducer 20' zusammen mit der Signalleitung 22' aus dem Lumen des Elektrodenleitungskörpers 12' entfernt werden kann. Die Signalleitung 22' ist dazu in den mit gleicher Bezugsziffer 22' bezeichneten Mandrin eingebettet. Dieser Mandrin 22' kann auch mehrere Signalleitungen enthalten und kann daher auch als Signalleitungskörper bezeichnet werden.

Allen Ausführungsvarianten ist gemein, dass die Hirnstimulationselektrodenleitung für die Deep Brain Stimulation einen oder mehrere Ultraschalltransducer entweder flächig oder auf einer Halbkugel im Sondenkopf der Hirnstimulationselektrodenleitung selbst oder an der Spitze eines Führungskatheters so angeordnet hat, dass die Erfassungsbereiche der Ultraschalltransducer in distale Richtung der Hirnstimulationselektrodenleitung voraus ausgerichtet sind.

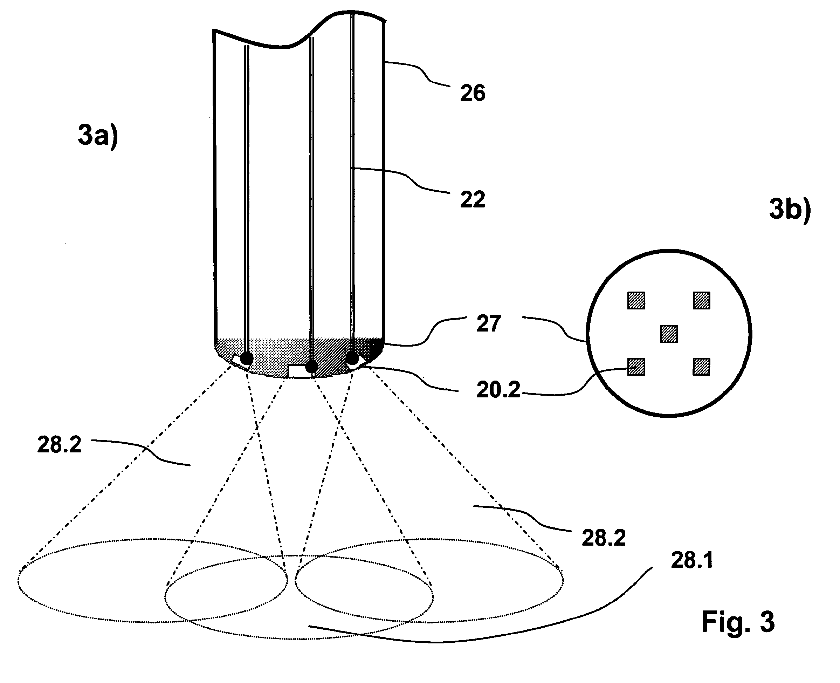

Figuren 3a und 3b zeigen eine mögliche Anordnung von 5 Ultraschallwandlerkristallen 20 als Ultraschalltransducer, die in die Spitze einer Hirnstimulationselektrodenleitung 10 auf einem konvexen Träger 27 so angeordnet sind, dass ein Recht- und Schrägvorausblick der jeweiligen Ultraschalltransducer 20.1 und 20.2 möglich ist.

Fig. 3a zeigt hierbei das distale Ende einer bevorzugten Ausführungsvariante eines solchen Signalleitungskörpers 26 mit darin eingebetteten Signalleitungen 22', die an ihrem jeweiligen distalen Ende mit einem jeweiligen Ultraschalltransducer 20" verbunden sind. Die Ultraschalltransducer 20" sind an einer Stirnfläche des Signalleitungskörpers 26 so eingebettet, dass sie in einer Ansicht auf die Stirnfläche (Fig. 3b) einen zentralen Ultraschalltransducer 20.1" sowie vier gleichmäßig darum verteilte periphere Ultraschalltransducer 20.2" bilden.

Wie Fig. 3a zu entnehmen ist, ist der zentrale Ultraschalltransducer 20.1" so ausgerichtet, dass sein Erfassungsbereich 28.1 konzentrisch zur verlängerten Längsachse des Signalleitungskörpers 26 ausgerichtet ist, während die peripheren Ultraschalltransducer 20.2" so angeordnet sind, dass ihre Erfassungsbereiche 28.2 jeweils schräg nach vorne ausgerichtet sind.

Der Signalleitungskörper 26 mit den insgesamt fünf Ultraschalltransducern 20.2' kann, wie in Figuren 2a und 2b dargestellt, in das Lumen eines Elektrodenleitungskörpers 12' so eingesetzt werden, dass die konvexe Stirnfläche des Signalleitungskörpers 26 bündig an einer entsprechenden konkaven Innenfläche eines entsprechend geformten Abschlusskörpers bündig anliegt. Auf diese Weise entsteht eine Hirnstimulationselektrodenleitung 10', die fünf Ultraschallerfassungskanäle aufweist.

Die Ultraschalltransducer 20 sind jeweils Pulswellendopplersensoren (Pulse Wave Doppler), die ausgebildet sind, bewegte Erythrozyten nach dem an sich bekannten Dopplerprinzip nachzuweisen. Auf diese Weise können mit Hilfe der Ultraschalltransducer 20 Blutgefäße im Erfassungsbereich eines jeweiligen Ultraschalltransducers 20 erkannt werden.

Dies erlaubt ein Erkennen von Blutgefäßen vor dem distalen Ende der Hirnstimulationselektrodenleitung 10, wenn diese zu einem jeweiligen Stimulationsort eingeführt werden soll.

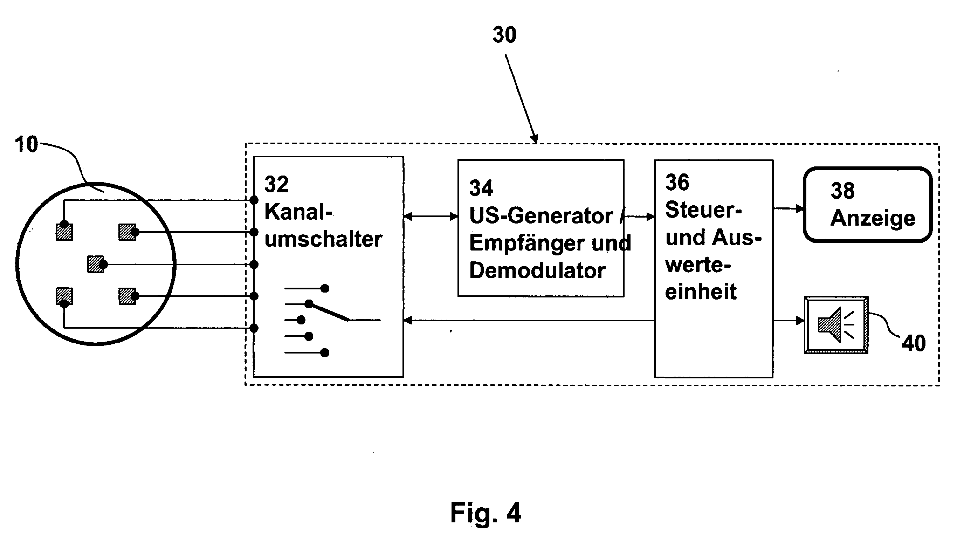

Hierzu sind die Ultraschalltransducer 20 der Hirnstimulationselektrodenleitung 10 mit einer Einführvorrichtung verbunden, die schematisch in Fig. 4 dargestellt ist und neben der Hirnstimulationselektrodenleitung 10 eine Signalverarbeitungseinrichtung 30 aufweist. Diese umfasst zum einen einen Kanalumschalter 32, um die von einem jeweiligen Ultraschalltransducer 20 stammenden Signale wahlweise einem Ultraschallgenerator und - empfänger sowie Demodulator 34 zuzuführen, dessen Ausgangswerte wiederum einer Steuer- und Auswerteeinheit 36 zugeführt werden. Diese Steuer- und Auswerteeinheit 36 steuert im Übrigen den Kanalumschalter 32. Außerdem ist die Steuer- und Auswerteeinheit 36 mit einer grafischen Anzeige 38 sowie mit einem akustischen Signalgeber 40 verbunden.

Der Ultraschallgenerator 34 erzeugt Ultraschallimpulse, deren Frequenz und Ultraschallenergie für eine intracerebrale Anwendung derart angepasst ist, dass in einem Abstand von 0-50 mm vor dem distalen Ende der Hirnstimulationselektrodenleitung 10 die Beurteilung eines dort verlaufenden Blutgefäßes möglich ist.

Die Steuer- und Auswerteeinheit 36 ist in Verbindung mit der optischen Anzeige 38 bzw. dem akustischen Signalgeber 40 so ausgebildet, dass das jeweils angezeigte bzw. akustisch wiedergegebene Signal unterschiedlich ist, je nachdem wie groß die Entfernung zwischen einem erfassten Blutgefäß und einem jeweiligen Ultraschalltransducer 20 ist. Das akustische wiedergegebene Signal kann beispielsweise in seiner Lautstärke vom Abstand abhängen. Besser noch hängt das akustische Signal hinsichtlich der Tonhöhe vom Abstand ab, wobei hohe Töne einen geringen Abstand kennzeichnen und niederfrequente Töne einen größeren Abstand. Überhaupt kein akustisches Signal zeigt an, dass sich im Erfassungsbereich des Ultraschalltransducers oder der Ultraschalltransducer kein Blutgefäß befindet.

Wenn die Signalverarbeitungseinrichtung 30 ein Blutgefäß in der Vorausrichtung eines Ultraschalltransducers erkennt, bestimmt die Steuer- und Auswerteeinheit 36 den Abstand zu diesem Blutgefäß und initiiert ein optisches und ein akustisches Signal, mit dem dem Anwender der Abstand zu dem erfassten Blutgefäß mitgeteilt wird. Die optische Anzeige 38 liefert neben der Abstandsinformation auch eine Information über die Richtung, in der ein Blutgefäß erfasst wurde, indem für jeden Ultraschalltransducer 20 ein separates optisches Anzeigeelement, beispielsweise in Form eines Sektors der optischen Anzeige 38 (siehe weiter unten) vorgesehen ist. Dieses Anzeigeelement wird beispielsweise entsprechend des Abstandes grün (kein Blutgefäß voraus), gelb (Blutgefäß in Abstand von a-b mm voraus), rot (Blutgefäß unmittelbar voraus) eingefärbt.

Ähnlich kann auch der akustische Signalgeber 40 so angesteuert werden, dass er eine Information über die Richtung enthält, in der ein Blutgefäß erfasst wurde: Ein unterbrochener akustischer Signalton wird in seiner Wiederholfrequenz umgekehrt proportional zum Abstand des Blutgefäßes gesteigert. Ist das Blutgefäß unmittelbar voraus, dann wird ein Dauerton abgegeben.

Der Anwender kann in der Auswerteeinheit 36 die Bereiche für die verschiedenen Warnstufen (grün, gelb, rot) individuell festlegen.

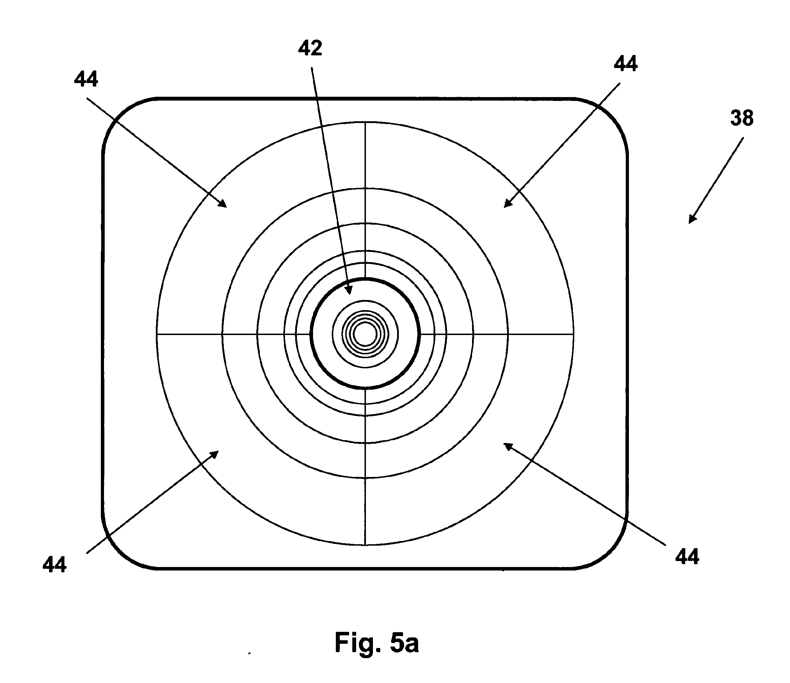

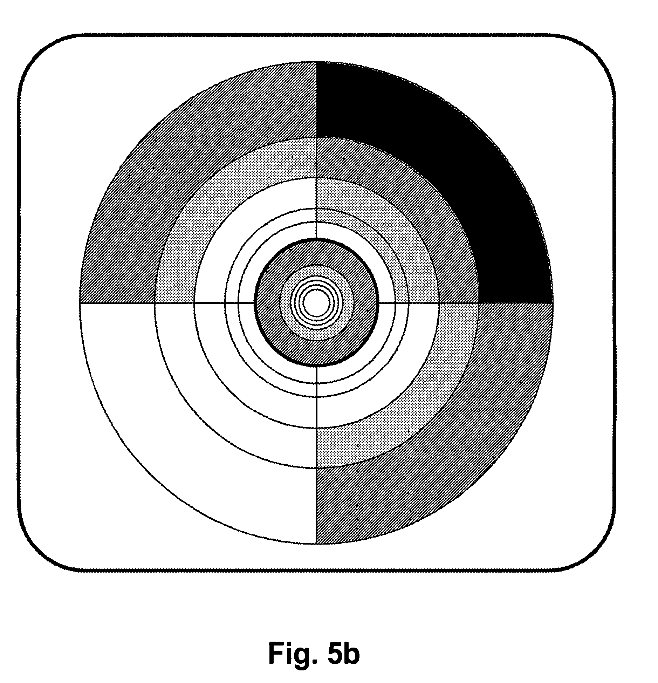

Die optische Anzeige 38 ist vorzugsweise wie in Fig. 5 dargestellt aufgebaut. Sie besitzt einen zentralen Sektor 42, der dem zentralen Transducer 20.1 zugeordnet ist und insgesamt vier periphere Sektoren 44, die jeweils einem der peripheren Transducer 20.2 zugeordnet sind. Die Sektoren sind jeweils in Untersektoren eingeteilt und die optische Anzeige 38 ist in Verbindung mit der Steuer- und Auswerteeinheit 36 so ausgebildet, dass die Zahl der angesteuerten Untersektoren größer ist, je näher ein jeweils erfasstes Blutgefäß dem jeweiligen Transducer ist. Ein gerade in dem Erfassungsbereich des zentralen Ultraschalltransducers 20.1 geratenes Blutgefäß führt dazu, dass nur ein zentraler Untersektor des zentralen Sektors 42 angesteuert, also beispielsweise schwarz dargestellt, wird. Je näher das erfasste Blutgefäß dem zentralen Ultraschalltransducer 20.1 ist, umso mehr Untersektoren des zentralen Sektors 42 werden angesteuert.

Entsprechend ist die Steuer- und Auswerteeinheit 36 in Verbindung mit der optischen Anzeige 38 ausgebildet, auch die Untersektoren der peripheren Sektoren 44 in Abhängigkeit des Abstandes zwischen einem jeweils erfassenden Ultraschalltransducer und einem Blutgefäß anzusteuern. Befindet sich das erfasste Blutgefäß noch in großer Entfernung zum jeweiligen Ultraschalltransducer 20.2, wird nur der innere Untersektor eines jeweiligen peripheren Sektors 44 angesteuert. Mit zunehmender Nähe werden nach und nach von innen nach außen weitere Untersektoren des peripheren Sektors 44 eingeschaltet, also beispielsweise dunkel dargestellt, wie dies beispielhaft in Figur 5b dargestellt ist. Eine derartige Anzeige erlaubt eine intuitive Einschätzung der jeweils vor dem distalen Ende der Hirnstimulationselektrodenleitung herrschenden Situation.

In Figur 5b ist die mögliche Darstellung der Abstandsinformation mit der optischen Anzeige 38 mit 5 Sektoren (4 Quadranten = schräg-voraus, 1 Zentrum = rechts-voraus) gemäß Figur 5a dargestellt. Jeder Sektor ist in Untersektoren unterteilt und zeigt mit den Farben weiß = kein Dopplersignal, grün = schwaches Signal, gelb = mittleres Signal, rot = starkes Signal den Abstand zu einem in der Richtung des dem jeweiligen Sektor 42 oder 44 der Anzeige 38 zugeordneten Ultraschalltransducers voraus liegendes Blutgefäß an.

## Patentansprüche

1. Hirnstimulationselektrodenleitung (10) für die elektrische Stimulation tief liegender Hirnbereiche mit einem längsgestreckten biegsamen Elektrodenleitungskörper (12), der an seinem distalen Ende oder in der Nähe seines distalen Endes wenigstens eine Stimulationselektrode (18) trägt, die zur Abgabe elektrischer Impulse an im Einsatzfall umliegendes Körpergewebe ausgebildet ist,
wobei der Elektrodenleitungskörper an seinem distalen Ende wenigstens einen Ultraschalltransducer (20) aufweist, der so angeordnet ist, dass er reflektierten Ultraschall in einem Erfassungsbereich erfassen kann, der in distaler Richtung entlang der Längsrichtung des Elektrodenleitungskörpers (12) ausgerichtet ist, und der ausgebildet ist, für interkraniale Blutgefäße charakteristische Reflektionen von Ultraschellwellen zu erfassen,
**dadurch gekennzeichnet, dass** der Elektrodenleitungskörper (12) ein Lumen aufweist, in das der Ultraschalltransducer entfernbar eingesetzt ist und dass das Lumen in dem Elektrodenleitungskörper an einem distalen Ende durch einen Abschlusskörper (24) verschlossen ist, der aus einem Material besteht, welches eine Ein- und Auskopplung sowie Übertragung von Ultraschallwellen mit geringer Dämpfung erlaubt und welcher auf einer zum Lumen weisenden Innenseite eine Form besitzt, die der Form des Ultraschalltransducers an dessen distalem Ende entspricht.

2. Hirnstimulationselektrodenleitung nach Anspruch 1, **dadurch gekennzeichnet, dass** der Ultraschalltransducer (20) mit wenigstens einer elektrischen Leitung verbunden ist, die als Signalleitung (22) zum proximalen Ende der Stimulationselektrodenleitung geführt ist und zum Übertragen solcher Erfassungssignale zu einem Anschluss an einem proximalen Ende der Hirnstimulationselektrodenleitung (10) ausgebildet ist, die von einem jeweiligen Ultraschalltransducer im Falle des Empfangs reflektierter Ultraschallwellen erzeugt werden.

3. Hirnstimulationselektrodenleitung nach Anspruch 1 oder 2, **dadurch gekennzeichnet, dass** der Ultraschalltransducer (20) fest mit dem Elektrodenleitungskörper (12) verbunden ist und im Bereich von dessen distalem Ende angeordnet ist.

4. Hirnstimulationselektrodenleitung (10) nach Anspruch 1, **dadurch gekennzeichnet, dass** die Signalleitungen Bestandteil eines fest mit dem Ultraschalltransducer verbundenen Leitungskörpers sind, der innerhalb des Lumens des Elektrodenleitungskörpers längsbeweglich ist.

5. Hirnstimulationselektrodenleitung nach einem der Ansprüche 1 bis 4, **dadurch gekennzeichnet, dass** die Hirnstimulationselektrodenleitung maximal 15 Ultraschalltransducer im Bereich ihres distalen Endes aufweist.

6. Hirnstimulationselektrodenleitung nach einem der Ansprüche 1 bis 5, **dadurch gekennzeichnet, dass** der Erfassungsbereich aller Ultraschalltransducer in distaler Richtung des Elektrodenleitungskörpers ausgerichtet ist und/oder dass der Ultraschalltransducer ein Pulse Wave Doppler Ultraschalltransducer ist, der zum Nachweis bewegter Erythrozyten ausgebildet ist und/oder dass der oder die Ultraschalltransducer mit elektrischen Leitungen verbunden sind, die als Signalleitungen zum proximalen Ende der Stimulationselektrodenleitung geführt sind und zum Übertragen von Erfassungssignalen ausgebildet sind, die von einem jeweiligen Ultraschalltransducer im Falle des Empfangs reflektierter Ultraschallwellen erzeugt werden.

7. Hirnstimulationselektrodenleitung nach einem der Ansprüche 1 bis 6, **dadurch gekennzeichnet, dass** der Elektrodenleitungskörper (12) auf seiner für die Implantation bestimmten Länge einen maximalen Außendurchmesser von 1 mm hat.

8. Einführvorrichtung für Hirnstimulationselektrodenleitungen gemäß eines der Ansprüche 1 bis 7, mit einer Hirnstimulationselektrodenleitung (10) gemäß eines der Ansprüche 1 bis 6 sowie mit einer Signalverarbeitungseinheit (30), die wenigstens indirekt mit dem Ultraschalltransducer (20) oder den Ultraschalltransducern (20.1, 20.2) der Hirnstimulationselektrodenleitung (10; 10') verbunden ist und die ausgangsseitig mit einer Anzeigeeinheit (38, 40) verbunden ist, die über den Ultraschalltransducer erfassten Signale anzuzeigen.

9. Einführvorrichtung gemäß Anspruch 8, **dadurch gekennzeichnet, dass** die Steuer- und Auswerteeinheit (36) in Verbindung mit der Anzeigeeinheit (38, 40) so ausgebildet ist, dass das angezeigte oder akustisch wiedergegebene Signal je nach Abstand eines erfassten Blutgefäßes vom jeweiligen Ultraschalltransducer (20) unterschiedlich ist.

10. Einführvorrichtung gemäß Anspruch 9, **dadurch gekennzeichnet, dass** die Anzeigeeinheit eine optische Anzeige (38) umfasst, die mit der Steuer- und Auswerteeinheit (36) verbunden und in Kombination mit dieser so ausgebildet ist, dass die Entfernung eines erfassten Blutgefäßes zum jeweiligen Ultraschalltransducer (20) durch unterschiedliche Farben dargestellt wird und/oder dass die Anzeigeeinheit einen akustischen Signalgeber (40) umfasst, der mit der Steuer- und Auswerteeinheit (36) verbunden und in Kombination mit dieser so ausgebildet ist, das unterschiedliche Entfernungen zwischen einem erfassten Blutgefäß und einem jeweiligen Ultraschalltransducer (20) durch unterschiedliche Tonhöhen wiedergegeben werden.

11. Einführvorrichtung gemäß eines der Ansprüche 8 bis 10, **dadurch gekennzeichnet, dass** die Signalverarbeitungseinheit (30) einen Kanalumschalter (32) sowie einen Ultraschallgenerator und -empfänger (34) umfasst, wobei der Kanalumschalter (32) mit den Ultraschalltransducern (20) und dem Ultraschallgenerator und - empfänger (34) derart verbunden ist, dass der Ultraschallgenerator und -empfänger (34) mit jeweils einem der Ultraschalltransducer gesteuert zu verbinden ist.

12. Einführvorrichtung gemäß eines der Ansprüche 8 bis 11, **dadurch gekennzeichnet, dass** der Ultraschallgenerator und -empfänger (34) ausgebildet ist, Ultraschallimpulse zu generieren und zu empfangen, deren Frequenz und Ultraschallenergie für eine intracerebrale Anwendung derart angepasst ist, dass in einem Abstand von 0-50 mm vor dem distalen Ende der Hirnstimulationselektrodenleitung 10 die Beurteilung eines dort verlaufenden Blutgefäßes möglich ist und/oder dass die Hirnstimulationselektrodenleitung (10) eine Mehrzahl von Ultraschalltransducern aufweist und die Steuer- und Auswerteeinheit (36) in Verbindung mit der Anzeigeeinheit (38, 40) ausgebildet ist, ein Signal zu generieren, welches die Lage eines erfassten Blutgefäßes relativ zur verlängerten Längsachse des distalen Endes des Elektrodenleitungskörpers wiedergibt.

13. Einführvorrichtung gemäß Anspruch 10 und 12, **dadurch gekennzeichnet, dass** die optische Anzeige (38) so aufgebaut ist dass sie in mehrere Anzeigesektoren (42, 44) unterteilt ist, von denen ein jeweiliger Sektor jeweils einem Ultraschalltransducer (20) zugeordnet ist, wobei die Anzeigesektoren (42, 44) in Untersektoren unterteilt sind und die Steuer- und Auswerteeinheit (36) in Verbindung mit der optischen Anzeige (38) ausgebildet ist, die Untersektoren in Abhängigkeit des Abstandes zu einem erfassten Blutgefäß anzusteuern, den die Steuer- und Auswerteeinheit (36) für einen jeweiligen Ultraschalltransducer bestimmt hat.

## Claims

1. A brain stimulation electrode line (10) for electrically stimulating deeper areas of the brain, comprising an elongated flexible electrode line body (12), which at the distal end or in the vicinity of the distal end carries at least one stimulation electrode (18) designed to deliver electrical impulses to surrounding body tissue during use,
the electrode line body at the distal end comprising at least one ultrasonic transducer (20), which is disposed such that it can capture reflected ultrasound in a capturing region oriented in the distal direction along the longitudinal direction of the electrode line body (12), and which is designed to capture reflections of ultrasonic waves that are characteristic of intracranial blood vessels, **characterized in that** the electrode line body (12) comprises a lumen into which the ultrasonic transducer is removably inserted, and the lumen in the electrode line body is closed at a distal end by a closing body (24), which comprises a material that allows ultrasonic waves to be coupled in and out and transmitted with little attenuation and which, on an inside directed toward the lumen, has a shape that corresponds to the shape of the ultrasonic transducer at the distal end.

2. The brain stimulation electrode line according to claim 1, **characterized in that** the ultrasonic transducer (20) is connected to at least one electrical line, which is guided as a signal line (22) to the proximal end of the stimulation electrode line and designed to transmit those captured signals which are generated by a respective ultrasonic transducer if reflected ultrasonic waves are received to a terminal at a proximal end of the brain stimulation electrode line (10).

3. The brain stimulation electrode line according to claim 1 or 2, **characterized in that** the ultrasonic transducer (20) is rigidly connected to the electrode line body (12) and disposed in the region of the distal end thereof.

4. The brain stimulation electrode line (10) according to claim 1, **characterized in that** the signal lines are part of a line body that is rigidly connected to the ultrasonic transducer, the body being longitudinally movable inside the lumen of the electrode line body.

5. A brain stimulation electrode line according to any one of claims 1 to 4, **characterized in that** the brain stimulation electrode line comprises no more than 15 ultrasonic transducers in the region of the distal end.

6. A brain stimulation electrode line according to any one of claims 1 to 5, **characterized in that** the capturing region of all ultrasonic transducers is oriented in the distal direction of the electrode lead body and/or the ultrasonic transducer is a pulse wave Doppler ultrasonic transducer, which is designed to detect moving erythrocytes, and/or the ultrasonic transducer or transducers are connected to electrical lines, which are guided as signal lines to the proximal end of the stimulation electrode line and designed to transmit captured signals, which are generated by a respective ultrasonic transducer if reflected ultrasonic waves are received.

7. A brain stimulation electrode line according to any one of claims 1 to 6, **characterized in that** the electrode line body (12) has a maximum outside diameter of 1 mm on the length thereof intended for implantation.

8. An insertion device for brain stimulation electrode lines according to any one of claims 1 to 7, comprising a brain stimulation electrode line (10) according to any one of claims 1 to 6, and comprising a signal processing unit (30), which is connected at least indirectly to the ultrasonic transducer (20) or the ultrasonic transducers (20.1, 20.2) of the brain stimulation electrode line (10; 10') and which, on the output side, is connected to a display unit (38, 40) to display the signals captured by the ultrasonic transducer.

9. The insertion device according to claim 8, **characterized in that** the control and evaluation unit (36), in conjunction with the display unit (38, 40), is designed such that the displayed or acoustically reproduced signal is different depending on the distance of a captured blood vessel from the respective ultrasonic transducer (20).

10. The insertion device according to claim 9, **characterized in that** the display unit comprises an optical display (38), which is connected to the control and evaluation unit (36) and in combination therewith is designed such that the distance of a captured blood vessel from the respective ultrasonic transducer (20) is represented by different colors and/or the display unit comprises an acoustic signal transmitter (40), which is connected to the control and evaluation unit (36) and in combination therewith is designed such that different distances between a captured blood vessel and a respective ultrasonic transducer (20) are reproduced by different tone pitches.

11. An insertion device according to any one of claims 8 to 10, **characterized in that** the signal processing unit (30) comprises a channel switching element (32) and an ultrasound generator and receiver (34), wherein the channel switching element (32) is connected to the ultrasonic transducers (20) and to the ultrasound generator and receiver (34) such that the ultrasonic generator and receiver (34) can be connected respectively in a controlled manner to one of the ultrasonic transducers.

12. An insertion device according to any one of claims 8 to 11, **characterized in that** the ultrasound generator and receiver (34) is designed to generate and receive ultrasonic pulses, the frequency and ultrasonic energy of which are adapted to an intracerebral application such that, at a distance of 0-50 mm in front of the distal end of the brain stimulation electrode line 10, a blood vessel located there can be assessed and/or the brain stimulation electrode line (10) comprises a plurality of ultrasonic transducers and the control and evaluation unit (36), in conjunction with the display unit (38, 40), is designed to generate a signal that reproduces the position of a captured blood vessel relative to the extended longitudinal axis of the distal end of the electrode line body.

13. An insertion device according to claims 10 and 12, **characterized in that** the optical display (38) is designed such that it is divided into a plurality of display sectors (42 to 44), each of which is associated with an ultrasonic transducer (20), wherein the display sectors (42, 44) are divided into sub-sectors, and the control and evaluation unit (36), in conjunction with the optical display (38), is designed to actuate the sub-sectors depending on the distance of a captured blood vessel, which was determined by the control and evaluation unit (36) for a particular ultrasonic transducer.

## Revendications

1. Ligne d'électrode de stimulation cérébrale (10), pour la stimulation électrique de régions du cerveau situées en profondeur, avec un corps de ligne d'électrode (12) flexible étendue dans la longueur portant au moins une électrode de stimulation (18) à son extrémité distale ou à proximité de son extrémité distale, celle-ci étant conçue pour délivrer des impulsions électriques au tissu corporel environnant pendant l'utilisation,
dans laquelle, à son extrémité distale, le corps de ligne d'électrode comporte au moins un transducteur à ultrason (20) disposé de manière à pouvoir capter l'ultrason réfléchi dans un périmètre de détection orienté en direction distale le long du sens longitudinal du corps de ligne d'électrode (12), et conçu pour détecter les réflexions d'ondes ultrasonores caractéristiques des vaisseaux sanguins inter-crâniens, **caractérisée en ce que** le corps de ligne d'électrode (12) présente un lumen dans lequel le transducteur à ultrason est placé de façon amovible, et **en ce que** le lumen est enfermé dans le corps de ligne d'électrode à une extrémité distale, par un corps de fermeture (24) constitué d'un matériau permettant un couplage et découplage ainsi que la transmission d'ondes ultrasonores avec un faible amortissement, et présentant, du côté intérieur tourné vers le lumen, une forme correspondant à la forme du transducteur à ultrason à l'extrémité distale de celui-ci.

2. Ligne d'électrode de stimulation cérébrale selon la revendication 1, **caractérisée en ce que** le transducteur à ultrason (20) est relié à au moins une ligne électrique, qui est conduite en tant que ligne de signal (22) jusqu'à l'extrémité proximale de la ligne d'électrode de stimulation et conçue pour la transmission de tels signaux de détection étant produits par un transducteur à ultrason respectif en cas de réception d'ondes ultrasonores réfléchies vers un raccord à une extrémité proximale de la ligne d'électrode à stimulation cérébrale (10).

3. Ligne d'électrode de stimulation cérébrale selon la revendication 1 ou 2, **caractérisée en ce que** le transducteur à ultrason (20) est relié solidement au corps de ligne d'électrode (12) et installé dans la région de l'extrémité distale de celui-ci.

4. Ligne d'électrode de stimulation cérébrale (10) selon la revendication 1, **caractérisée en ce que** les lignes de signaux sont des composants d'un corps de conducteur relié solidement au transducteur à ultrason, qui est déplaçable dans la longueur à l'intérieur du lumen du corps de ligne d'électrode.

5. Ligne d'électrode de stimulation cérébrale selon l'une des revendications 1 à 4, **caractérisée en ce que** la ligne d'électrode de stimulation cérébrale comporte au maximum 15 transducteurs à ultrason dans la région de son extrémité distale.

6. Ligne d'électrode de stimulation cérébrale selon l'une des revendications 1 à 5, **caractérisée en ce que** la zone de détection de tous les transducteurs à ultrason est orientée en direction distale du corps de ligne d'électrode et/ou **en ce que** le transducteur à ultrason est un transducteur à ultrason Pulse Wave Doppler, conçu pour la détection d'érythrocytes déplacés, et/ou **en ce que** le ou les transducteurs à ultrason sont reliés à des lignes électriques conduites en tant que lignes de signal jusqu'à l'extrémité proximale de la ligne d'électrode de stimulation et conçues pour la transmission de signaux de détection produites par un transducteur à ultrason respectif en cas de réception d'ondes ultrasonores réfléchies.

7. Ligne d'électrode de stimulation cérébrale selon l'une des revendications 1 à 6, **caractérisée en ce que** le corps de ligne d'électrode (12) présente un diamètre extérieur maximal de 1 mm sur sa longueur définie pour l'implantation.

8. Dispositif d'introduction pour des lignes d'électrode de stimulation cérébrale selon l'une des revendications 1 à 7, avec une ligne d'électrode de stimulation cérébrale (10) selon l'une des revendications 1 à 6 et une unité de traitement de signaux (30) reliée au moins indirectement au transducteur à ultrason (20) ou aux transducteurs à ultrason (20.1, 20.2) de la ligne d'électrode de stimulation cérébrale (10 ; 10'), et reliée côté sortie à une unité d'affichage (38, 40) conçue pour afficher les signaux détectés par le transducteur à ultrason.

9. Dispositif d'introduction selon la revendication 8, **caractérisé en ce que** l'unité d'évaluation et de commande (36) est conçue de telle manière en relation avec l'unité d'affichage (38, 40), que le signal affiché ou restitué acoustiquement diffère en fonction de la distance entre un vaisseau sanguin détecté et le transducteur à ultrason (20) respectif.

10. Dispositif d'introduction selon la revendication 9, **caractérisé en ce que** l'unité d'affichage comprend un indicateur optique (38) relié à l'unité d'évaluation et de commande (36) et conçu de telle manière, en combinaison avec celle-ci, que la distance entre un vaisseau sanguin détecté et le transducteur à ultrason (20) respectif est représentée par différentes couleurs, et/ou **en ce que** l'unité d'affichage comprend un indicateur de signal sonore (40) relié à l'unité d'évaluation et de commande (36) et conçu de telle manière, en combinaison avec celle-ci, que des distances différentes entre un vaisseau sanguin détecté et un transducteur à ultrason (20) respectif est restituée par différents niveaux sonores.

11. Dispositif d'introduction selon l'une des revendications 8 à 10, **caractérisé en ce que** l'unité de traitement de signaux (30) comprend un commutateur de canal (32) ainsi qu'un générateur-récepteur d'ultrasons (34), le commutateur de canal (32) étant relié de telle manière aux transducteurs à ultrason (20) et au générateur-récepteur d'ultrasons (34), que le générateur-récepteur d'ultrasons (34) peut être relié respectivement à l'un des transducteurs à ultrason de façon commandée.

12. Dispositif d'introduction selon l'une des revendications 8 à 11, **caractérisé en ce que** le générateur-récepteur d'ultrasons (34) est conçu pour générer et recevoir des impulsions ultrasonores, dont la fréquence et l'énergie ultrasonique sont adaptées de telle manière à une application inter-cérébrale, que sur une distance de 0-50 mm avant l'extrémité distale de la ligne d'électrode de stimulation cérébrale (10), il est possible de d'évaluer un vaisseau sanguin passant à cet endroit, et/ou **en ce que** la ligne d'électrode de stimulation cérébrale (10) comporte une multitude de transducteurs à ultrason, et l'unité d'évaluation et de commande (36) en relation avec l'unité d'affichage (38, 40) est conçue pour générer un signal restituant la position d'un vaisseau sanguin détecté par rapport à l'axe longitudinal prolongé de l'extrémité distale du corps de ligne d'électrode.

13. Dispositif d'introduction selon les revendications 10 et 12, **caractérisé en ce que** l'indicateur optique (38) est construit de manière à être divisée en plusieurs secteurs d'indication (42, 44), parmi lesquels un secteur est à chaque fois respectivement attribué à un transducteur à ultrason (20), les secteurs d'indication (42, 44) étant divisés en sous-secteurs, et l'unité d'évaluation et de commande (36) en relation avec l'unité d'affichage (36) étant conçue pour commander les sous-secteurs en fonction de la distance par rapport à un vaisseau sanguin détecté que l'unité d'évaluation et de commande a déterminée pour un certain transducteur à ultrason.
